# EUROPEAN PATENT APPLICATION

(11) **EP 2 495 237 A1**
(43) Date of publication of application: **05.09.2012**
(21) Application number: 12157420.6
(22) Date of filing: 29.02.2012
(51) Int. Cl.: C07D 223/16

(54) **An improved process for the preparation of highly pure ivabradine hydrochloride**

(30) Priority: 04.03.2011 IN BO06142011
(71) Applicant: Alembic Pharmaceuticals Limited, Vadodara 390 003 (IN)
(72) Inventor: Raman, Jayaraman, Venkat, 390003 Vadodara (IN); Tomer, Sanjiv, 390003 Vadodara (IN); Rana, Piyush, 390003 Vadodara (IN); Kanzariya, Kamlesh, 390003 Vadodara (IN); Borsaniya, Manoj, 390003 Vadodara (IN)
(74) Representative: Watson, Robert James

(57) **Abstract**

The present invention encompasses a process for the preparation of highly pure Ivabradine hydrochloride by treating crude Ivabradine with derivatizing agent which derivatize impurities adhered with the crude Ivabradine.

## Description

### FIELD OF THE INVENTION

The present invention encompasses a process for the preparation of highly pure Ivabradine hydrochloride by treating crude Ivabradine with a derivatizing agent which derivatizes impurities adhered with the crude Ivabradine.

### BACKGROUND OF THE INVENTION

Ivabradine hydrochloride of formula I, has very valuable pharmacological and therapeutic properties, and is useful in many cardiovascular diseases such as angina pectoris, myocardial infarct and associated rhythm disturbances and is chemically known as (S)-7,8-dimethoxy-3-{3-{N-[(4,5-dimethoxybenzocyclobut-1-yl)methyl]-*N-*(methyl)amino)propyl)-1,3,4,5-tetrahydro-2H-3-benzazepin-2-one hydrochloride.

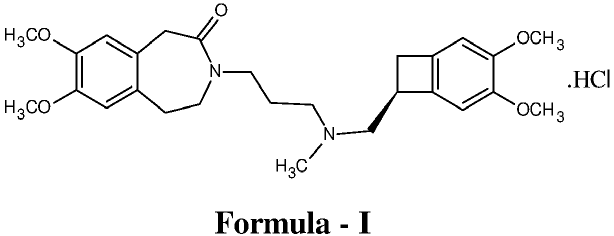

Ivabradine with a pharmaceutically acceptable acid have very valuable pharmacological and therapeutic properties, especially bradycardic properties, making those compounds useful in the treatment or prevention of various clinical situations of myocardial ischemia such as angina pectoris, myocardial infarct and associated rhythm disturbances, and also in various pathologies involving rhythm disturbances, especially supraventricular rhythm disturbances, and in heart failure.

Ivabradine hydrochloride is first disclosed in U.S. Patent No. 5,296,482. The disclosed process comprises the condensation of (S)-N-[(4,5-dimethoxybenzocyclobut-1-yl)-methyl]-*N*(methyl)amine of Formula II, 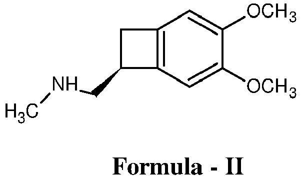 with 7,8-dimethoxy-3-[3-iodopropyl]-1,3-dihydro-2H-3-benzazepin-2-one Formula VII, 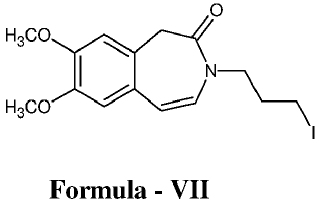 in acetone and in presence of a base such as potassium carbonate. The resulting benzazepine intermediate of formula IV is purified by column chromatography, and 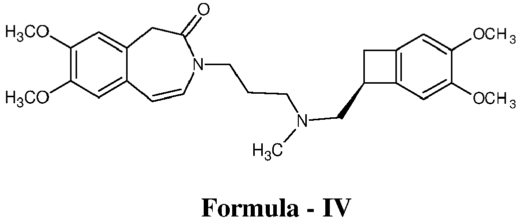 is further reduced with palladium hydroxide in glacial acetic acid, under the atmosphere of hydrogen gas to get ivabradine which is converted into its hydrochloride salt by the action of aqueous hydrochloric acid.

The methyl amine derivative of formula II is prepared by the reduction of 1-cyano 4,5dimethoxybenzocyclobutane of Formula V, 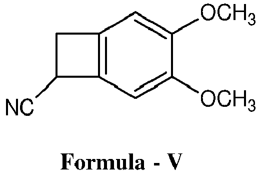 with borane tetrahydrofuran complex which upon condensation with ethyl chloroformate and further reducion with lithium aluminium hydride in tetrahydrofuran resulted in racemic compound of methyl amine derivative of formula II. The racemic compound is resolved to (+) isomer of methyl amine derivative of formula II with (d)-camphosulphonic acid.

Benzazepine derivative of formula VI is prepared by the reaction of sodium iodide with 7,8-dimethoxy-3-[3-chloropropyl]-1,3-dihydro-2H-3-benzazepin-2-one of formula VIIa, 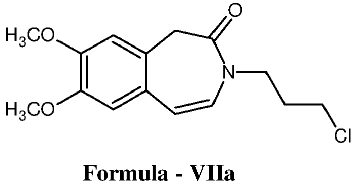 in acetone and the resulting iodo intermediate is purified by dissolving it in water and extraction with dichloromethane.

It has been observed that the said process suffers from many drawbacks such as the use of borane-tetrahydrofuran complex which is unstable at room temperature and purification of intermediates and ivabradine by chromatographic techniques. The chromatographic technique for purification is cumbersome, tedious and difficult to utilize on an industrial scale.

Several methods for the preparation of Ivabradine have been described. Like any synthetic compound, Ivabradine, or a pharmaceutically-acceptable salt thereof can contain process impurities, unreacted starting materials, chemical derivatives of impurities contained in starting materials, synthetic by-products, and degradation products. It is also known in the art that impurities present in an active pharmaceutical ingredient ("API") may arise from degradation of the API, for example, during storage or during the manufacturing process, including the chemical synthesis.

It is well known in the art that, for human administration, safety considerations require the establishment, by national and international regulatory authorities, of very low limits for identified, but toxicologically uncharacterized impurities, before an active pharmaceutical ingredient (API) product is commercialized. Typically, these limits are less than about 0.15 percent by weight of each impurity. Limits for unidentified and/or uncharacterized impurities are obviously lower, typically less than 0.1 percent by weight. Specific standards can be applied to certain drugs where a pharmacopoeia monograph has been established for that drug. Typically, for impurities that are present in an amount of greater than 0.1 percent by weight, the impurity should be fully identified and characterized.

Therefore, in the manufacture of active pharmaceutical ingredients (APIs) knowledge of the purity of the API, such as Ivabradine Hydrochloride, is required before commercialization, as is the purity of the API in the manufactured formulated pharmaceutical product.

Impurities introduced during commercial manufacturing processes must be limited to very small amounts and are preferably substantially absent. For example, the ICH Q7A guidance for API manufacturers requires that process impurities be maintained below set limits by specifying the quality of raw materials, controlling process parameters, such as temperature, pressure, time, and stoichiometric ratios, and including purification steps, such as crystallization, distillation, and liquid-liquid extraction, in the manufacturing process.

The direct product of a chemical reaction is rarely a single compound with sufficient purity to comply with pharmaceutical standards. Intermediates and by-products will, in most cases, be present with the API. At certain stages during processing of an API, such as Ivabradine, it must be analyzed for purity, typically, by HPLC or TLC analysis, to determine the presence of any intermediates or by-products. The API need not be absolutely pure, as absolute purity is a theoretical ideal that is typically unattainable. Rather, purity standards are set with the intention of ensuring that an API is as free of impurities as possible, and, thus, is as safe as possible for human use. As discussed above, in the United States, the Food and Drug Administration guidelines recommend that the amounts of some impurities be limited to less than 0.1 % wt.

Generally, by-products and intermediates (collectively hereinafter defined as "impurities") are identified spectroscopically and/or with another physical method, and then associated with a peak position, such as that in a chromatogram, or a spot on a TLC plate. [Strobel p. 953, Strobel, H. A.; Heineman, W.R., Chemical Instrumentation: A Systematic Approach, 3rd ed. (Wiley & Sons: New York 1989)]. Thereafter, the impurity can be identified, e.g., by its position in the chromatogram, where the position in a chromatogram is conventionally measured in minutes between injection of the sample on the column and elusion of the particular component through the detector.

Impurities generally found in pharmaceutically active agents and formulations containing them include residual amounts of synthetic precursors to the active agent, by-products which arise during synthesis of the active agent, residual solvent, isomers of the active agent, contaminants which were present in materials used in the synthesis of the active agent or in the preparation of the pharmaceutical formulation, and unidentified adventitious substances. Other impurities which may appear on storage include substances resulting from degradation of the active agent, for instance by oxidation or hydrolysis. The ICH Q7A guidance for API manufacturers requires that process impurities be maintained below set limits by specifying the quality of raw materials, controlling process parameters, such as temperature, pressure, time, and stoichiometric ratios, and including purification steps, such as crystallization, distillation, and liquid-liquid extraction, in the manufacturing process.

At certain stages during processing of an API, such as Ivabradine, or a pharmaceutically-acceptable salt thereof, it must be analyzed for purity, typically, by HPLC or GC analysis, to determine if it is suitable for continued processing and, ultimately, for use in a pharmaceutical product. The API need not be absolutely pure, as absolute purity is a theoretical ideal that is typically unattainable. Rather, purity standards are set with the intention of ensuring that an API is as free of impurities as possible, and, thus, are as safe as possible for clinical use. In the International Conference on Harmonisation of Technical Requirements for Registration of Pharmaceuticals for Human Use ("ICH") guidelines recommend that the amounts of unknown impurities be limited to less than 0.1 percent.

As is known by those skilled in the art, the management of process impurities is greatly enhanced by understanding their chemical structures and synthetic pathways, and by identifying the parameters that influence the amount of impurities in the final product.

The above mentioned drawbacks call for an alternative and improved process for the preparation of highly pure Ivabradine hydrochloride that would be commercially viable, reproducible on industrial scale and meets the needs of regulatory agencies.

In view of the above, it is, therefore, desirable to provide an efficient, more economical, less hazardous and eco-friendly process for the preparation of highly pure Ivabradine or a pharmaceutically acceptable salt thereof where impurity formation is less and hence avoids chromatographic purification and is convenient to operate on a commercial scale. Further, an amorphous form of Ivabradine hydrochloride has also been provided in the present application for which the protection is sought.

While developing a process for the preparation of Ivabradine hydrochloride, present inventors serendipitously found improved process for the preparation of highly pure Ivabradine which involves treating crude Ivabradine with derivatizing agent.

### OBJECT OF THE INVENTION

Therefore, it is an object of the present invention is to provide a process for the preparation of highly pure Ivabradine hydrochloride which comprising step of treating crude Ivabradine with a derivatizing agent in a suitable solvent.

Another object of the present invention is to provide a process for the preparation of highly pure Ivabradine hydrochloride which comprising step of treating crude Ivabradine with acetyl chloride in a suitable solvent.

Yet another aspect of the invention is to provide highly pure Ivabradine hydrochloride having purity at least about 99 % with the content of N-[(4,5-dimethoxybenzocyclobut-1-yl)-methyl]-7N-(methyl)amine of formula II in the range of about 0.03 % to about 0.15 % as measured by HPLC.

### SUMMARY OF THE INVENTION

According to one aspect of the present invention it provides a process for the preparation of highly pure Ivabradine hydrochloride comprising step of:
a) treating crude Ivabradine with a derivatizing agent in suitable solvent;
b) isolating highly pure Ivabradine hydrochloride.

According to another aspect of the present invention it provides a process for the preparation of highly pure Ivabradine hydrochloride comprising step of:
a) treating crude Ivabradine with acetyl chloride in suitable solvent.
b) isolating highly pure Ivabradine hydrochloride.

According to another aspect of the present invention it provides a process for the preparation of highly pure Ivabradine hydrochloride of formula I which comprises:
a) condensing methylamine derivative of formula II with 3-(3-chloropropyl)-7, 8-dimethoxy-1, 3, 4, 5-tetrahydro-2*H*-3-benzazepin-2-one of formula III 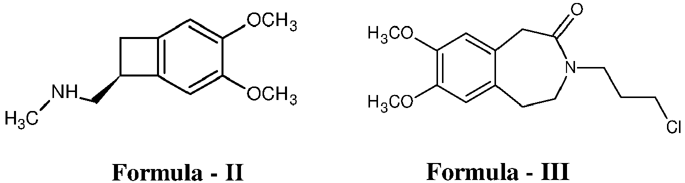 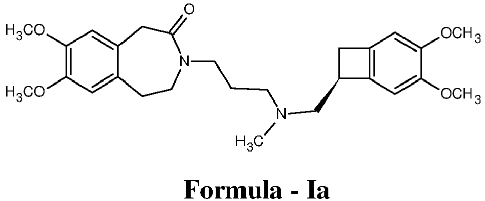
b) treating crude Ivabradine with derivatizing agent in suitable solvent.

According to another aspect of the present invention it provides a process for the preparation of highly pure Ivabradine hydrochloride comprising step of:
a) condensing methylamine derivative of formula II with 3-(3-chloropropyl)-7, 8-dimethoxy-1, 3, 4, 5-tetrahydro-2*H*-3-benzazepin-2-one of formula III 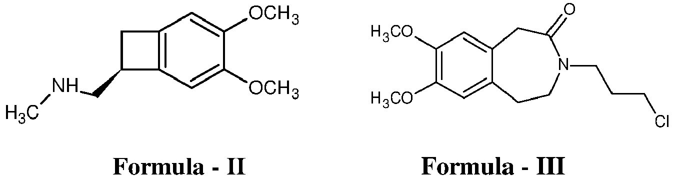 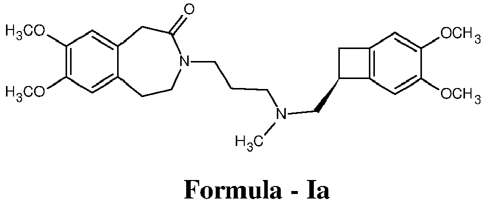
b) treating crude Ivabradine with acetyl chloride in suitable solvent;
c) treating Ivabradine with hydrochloride to obtain highly pure Ivabradine hydrochloride;
d) crystallizing highly pure Ivabradine hydrochloride with acetonitrile and methanol; and
e) isolating highly pure Ivabradine hydrochloride;

According to another aspect of the present invention it provides a process for the preparation of highly pure Ivabradine hydrochloride which comprises treating crude Ivabradine with a derivatizing agent in suitable solvent.

Yet another aspect of the invention is to provide highly pure Ivabradine hydrochloride having purity at least about 99 % with the content of N-[(4,5-dimethoxybenzocyclobut-1-yl)-methyl]-7N-(methyl)amine of formula II in the range of about 0.03% to about 0.15% as measured by HPLC.

### DETAILED DESCRIPTION OF THE INVENTION

As used herein, "highly pure Ivabradine hydrochloride" may refer to Ivabradine hydrochloride substantially free of N-[(4,5-dimethoxybenzocyclobut-l-yl)-methyl]-7N-(methyl)amine of formula II, an impurity of Ivabradine hydrochloride, which is present in an amount of less than of about 0.03 % to about 0.2 % as measured by HPLC. Specifically, the Ivabradine hydrochloride, as disclosed herein, may contain about 0.03% to about 0.15%, more specifically about 0.03 % to about 0.10 %, still more specifically less than about 0.03% to about 0.05% N-[(4,5-dimethoxybenzocyclobut-1-yl)-methyl]-7N-(methyl)amine of formula II impurity. In one embodiment, the highly pure Ivabradine hydrochloride disclosed herein comprises N-[(4,5-dimethoxybenzocyclobut-1-yl)-methyl]-7N-(methyl)amine of formula II impurity in an amount of less than about 0.15%, specifically in an amount of less than about 0.10%, more specifically in an amount of less than about 0.05%, as measured by HPLC. In another embodiment, the highly pure Ivabradine hydrochloride disclosed herein has a total purity of greater than about 99%, specifically greater than about 99.5%, more specifically greater than about 99.9%, and most specifically greater than about 99.95% as measured by HPLC. For example, the purity of the highly pure Ivabradine hydrochloride may be about 99% to about 99.9%, or about 99.5% to about 99.99%.

The term "Derivatizing agent" refers to agents which derivatize impurities adhered with the crude Ivabradine. The derivatizing agent may include, but is not limited to, an agent comprising any nitrogen or oxygen protecting group selected from the group consisting of Carbobenzyloxy (Cbz), p-Methoxybenzyl carbonyl (Moz or MeOZ), tert-Butyloxycarbonyl (BOC), 9-Fluorenylmethyloxycarbonyl (FMOC), Acetyl (Ac) group - Acetyl halide, sulfonyl group- p-Toluenesulfonic acid, *p*-toluenesulfonyl chloride, *p*-bromobenzenesulfonyl, 2- or 4-nitrobenzenesulfonyl, methanesulfonic acid (CH₃SO₃H), methanesulfonyl chloride, trifluoromethanesulfonyl, 5-(dimethylamino)naphthalene-1-sulfonyl. A most preferred derivatizing agent is acetyl chloride.

One embodiment of the present invention relates to an improved process for the preparation of highly pure Ivabradine hydrochloride by initially condensing methylamine derivative of formula II with benzazepine derivative of formula III to form crude Ivabradine of formula I in the presence of a base, in polar aprotic or protic solvents. The base can be selected from alkali metal carbonates, bicarbonates and hydroxides and preferably potassium carbonate. The polar aprotic or protic solvent may be selected from tetrahydrofuran, acetone, acetonitrile, dimethylformamide, dimethylsulfoxide, water, isopropanol, C₁-C₄ linear aliphatic alcohols such as methanol, ethanol etc. and mixtures thereof. More preferably, the solvent is dimethylformamide or dimethylsulfoxide and most preferably the solvent is dimethylformamide. It is advantageous to use dimethylformamide during condensation reaction because reaction rate is faster. Reaction is conducted at 25-95°C and preferably at 55-75°C. The completion of reaction can be monitored by high performance liquid chromatography. After completion of the reaction the reaction mass is charged with derivatizing agent, and the reaction mass is stirred for 2 to 10 hours. The reaction mass is than cooled with chilled water to ambient temperature. The product is extracted in organic solvent from aqueous layer and some impurities remain in the aqueous layer. The solvent can be selected from halogenated hydrocarbon such as methylene dichloride, ethylene dichloride, carbon tetrachloride, chloroform and aliphatic ester such as ethyl acetate and preferably ethyl acetate is used. Thereafter, solvent is distilled off completely and the product is purified by acid base wash treatment. Specifically the residue is treated with hydrochloric acid and washed with solvent such as acetonitrile. The product is obtained in high purity and no chromatographic purification is required.

It has also been found that during condensation process an unreacted process impurity is present, which is not easily removed by using the prior art isolation process and hence decreases the purity of the condensed product. But during the process of present invention, it is observed that impurity can be selectively removed by derivatizing with a derivatizing agent in suitable solvents such as toluene, tetrahydrofuran, acetone, ethyl acetate, triethylamine, acetonitrile, dimethylformamide, dimethylsulfoxide, water, isopropanol, methylene dichloride, ethylene dichloride, carbon tetrachloride, chloroform, and C₁-C₄ linear aliphatic alcohols such as methanol, ethanol and the like or mixtures thereof. More preferred solvents are toluene, triethylamine, dimethylformamide or dimethylsulfoxide. This further avoids the use of tedious chromatographic purification.

The complete representation of an embodiment of the process for the preparation of highly pure Ivabradine hydrochloride as provided by the present invention is depicted by the following schematic diagram:

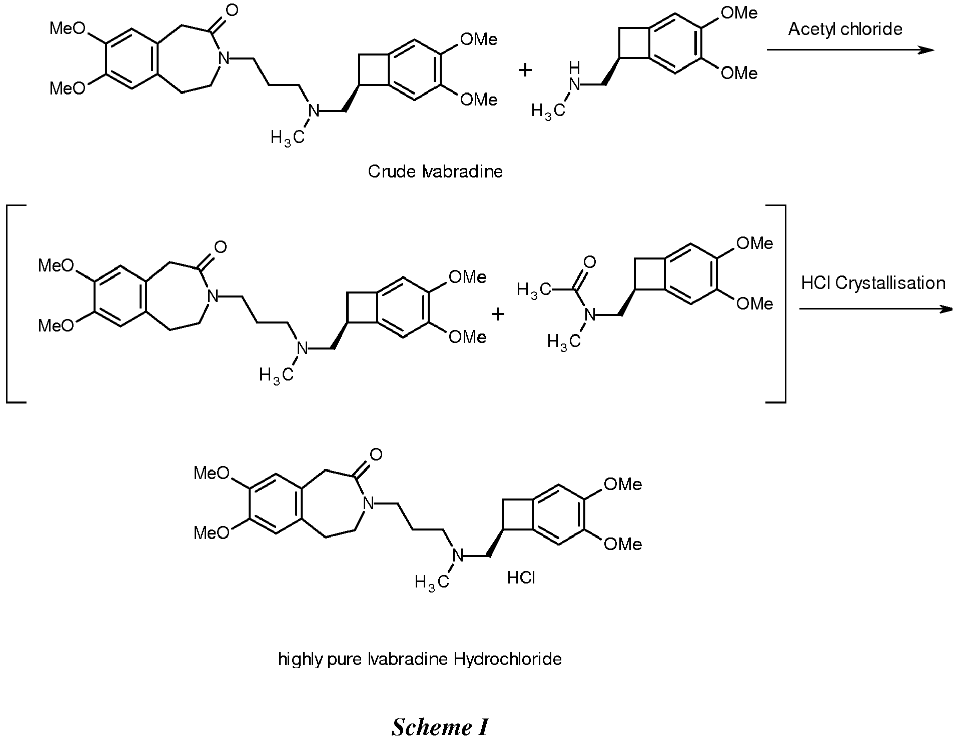

In another embodiment, the present invention provides a highly pure Ivabradine hydrochloride having purity at least about 99 % wherein the content of N-[(4,5-dimethoxybenzocyclobut-1-yl)-methyl]-7N-(methyl)amine of formula II impurity is in the range of about 0.03 % to about 0.15 % as measured by HPLC and at least one pharmaceutically acceptable excipient.

Comparison of the purification process of Ivabradine hydrochloride of priorart and present invention is mentioned in Table I.

**Table I**

| **Sr. No.** | **Experiment Details** | **Content of Intermediate-II as unreacted impurity in Ivabradine Hydrochloride** |
|---|---|---|
| 1 | Ivabradine Hydrochloride Crude | 1.41 |
| 2 | Purification with Acetonitrile(without derivatizing reaction) | 1.26 |
| 3 | Purification with Acetone (without derivatizing reaction) | 1.40 |
| 4 | Purification with Acetonitrile (after derivatizing reaction) | 0.12 |
| 5 | Purification with Acetone (after derivatizing reaction) | Nil |

This new method of purification by derivatizing was extensively studied using various reagents, bases and solvents. The most preferred results were obtained by treatment of the reaction mixture with acetyl chloride, followed by recrystallization. This novel method of Derivatization of the impurities present in crude Ivabradine allowed removal of the corresponding acetylated derivatives by recrystallization in solvent.

The process of the present invention has following advantages:
(i) It eliminates the requirement of chromatographic purification method of Ivabradine and provides a process which is economical, operational on and industrially applicable.
(ii) The process provides less number of purification steps.
(iii) The process is simple and easy to handle and does not require special handling care or critical temperature conditions.
(iv) It eliminates the use of reagents which is greatly air and moisture sensitive.

The following examples illustrate the invention further. The examples are set forth to aid in understanding the invention but are not intended to, and should not be construed to limit its scope in any way.

### Example 1

### Preparation of highly pure Ivabradine hydrochloride

Charge acetone (10ml), 3-(3-chloropropyl)-7,8-dimethoxy-1,3,4,5-tetrahydro-2*H*-3-benzazepin-2-one of formula IV (2.4gm), potassium carbonate (1.6gm), (S)-N-[(4,5-dimethoxybenzocyclobut-1-yl)-methyl]-7*N*-(methyl)amine (1.0 gm) and sodium iodide at room temperature. The reaction mixture was heated and stirred at 50-65 °C. After completion of reaction distill out Acetone atmospherically and separate the organic layer in toluene. Add triethylamine (0.12gm) reaction mass and further charged with acetyl chloride (0.08 gm). The reaction mass was than cooled in chilled water. The reaction mass was acidify with concentrated hydrochloride to pH 2-3 and the product is extracted in aqueous layer. The layers were separated and pH of the aqueous layer was adjusted to 10.5-12.5 with aqueous sodium hydroxide solution. Toluene was added to the aqueous layer and product was extracted in organic layer. Toluene was distilled out completely under vacuum to get residue of Ivabradine. Thereafter, the residue was taken in methanolic hydrochloride (10ml). The solvent was removed by distillation under reduced pressure. The product, thus obtained, was further stirred with Acetonitrile (10 ml) for 2-3 hours, filtered, and dried under vacuum to obtain Ivabradine hydrochloride having purity 99.9% area by High performance liquid chromatography (HPLC).

### Example 2

### Preparation of highly pure Ivabradine hydrochloride

Charge DMF (15 ml), 3-(3-chloropropyl)-7,8-dimethoxy-1,3,4,5-tetrahydro-2*H*-3-benzazepin-2-one of formula IV (1.0 gm), potassium carbonate (1.0 gm), (S)-N-[(4,5-dimethoxybenzocyclobut-1-yl)-methyl]-7*N*-(methyl)amine (0.45 gm) and sodium iodide at room temperature. The reaction mixture was heated and stirred at 60-65 °C. After completion of reaction, reaction mass add triethylamine (0.06 gm) and Carbobenzyloxy (Cbz) (0.08 gm) and stirred for 2-3 hours. The reaction mass was than cooled in chilled water. The reaction mass was acidify with concentrated hydrochloride to pH 2-3 and the product is extracted in ethyl acetate. The aqueous layer was further extracted with ethyl acetate (65ml). Combined ethyl acetate layers was acidified with aqueous hydrochloric acid and stirred. The layers were separated and pH of the aqueous layer was adjusted to 10.5-12.5 with aqueous sodium hydroxide solution. The aqueous layer is extracted with ethyl acetate (140ml + 60ml). Ethyl acetate was distilled out completely under vacuum to get residue of Ivabradine. Thereafter, the residue was taken in Isopropyl alcohol hydrochloride (10ml). The solvent was removed by distillation under reduced pressure. The product, thus obtained, was further stirred with acetone (10 ml) for 2-3 hours, filtered, and dried under vacuum to obtain Ivabradine hydrochloride having purity 99.9% area by High performance liquid chromatography (HPLC).

### Example 3

### Preparation of highly pure Ivabradine hydrochloride

Charge acetone (10 ml), 3-(3-chloropropyl)-7,8-dimethoxy-1,3,4,5-tetrahydro-2*H*-3-benzazepin-2-one of formula IV (1.0 gm), potassium carbonate (1.6 gm), (S)-N-[(4,5-dimethoxybenzocyclobut-1-yl)-methyl]-7*N*-(methyl)amine (1.8 gm) and sodium iodide at room temperature. The reaction mixture was heated and stirred at 50-65 °C. After completion of reaction distill out Acetone atmospherically and separate the organic layer in toluene. Add triethylamine (0.12gm) reaction mass and further charged with *p*-toluenesulfonyl chloride (0.19 gm). The reaction mass was than cooled in chilled water. The reaction mass was acidify with concentrated hydrochloride to pH 2-3 and the product is extracted in aqueous layer. The layers were separated and pH of the aqueous layer was adjusted to 10.5-12.5 with aqueous sodium hydroxide solution. Toluene was added to the aqueous layer and product was extracted in organic layer. Toluene was distilled out completely under vacuum to get residue of Ivabradine. Thereafter, the residue was taken in methanolic hydrochloride (10ml). The solvent was removed by distillation under reduced pressure. The product, thus obtained, was further stirred with acetone (10 ml) for 2-3 hours, filtered, and dried under vacuum to obtain Ivabradine hydrochloride having purity 99.9% area by High performance liquid chromatography (HPLC).

## Claims

1. A process for preparation of highly pure Ivabradine hydrochloride comprising:
a) treating crude Ivabradine with a derivatizing agent in suitable solvent;
b) isolating highly pure Ivabradine hydrochloride.

2. A process for preparation of highly pure Ivabradine hydrochloride according to claim 1, which comprising the additional step of:
preparing the crude Ivabradine of formula Ia by condensing the methylamine derivative of formula II with 3-(3-chloropropyl)-7, 8-dimethoxy-1, 3, 4, 5-tetrahydro-2*H*-3-benzazepin-2-one of formula III

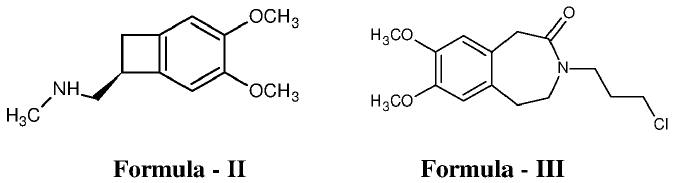

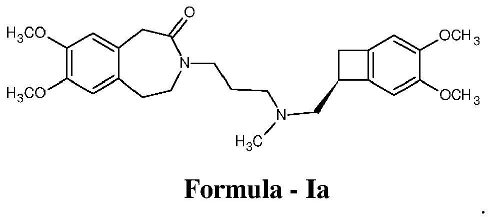

3. A process for preparation of highly pure Ivabradine hydrochloride according to either claim 1 or claim 2 whereinthe derivatizing agent comprises a nitrogen or oxygen protecting group.

4. A process for preparation of highly pure Ivabradine hydrochloride according to claim 3 wherein the derivatizing agent comprises a protecting group selected from the group consisting of Carbobenzyloxy (Cbz), p-Methoxybenzyl carbonyl (Moz or MeOZ), tert-Butyloxycarbonyl (BOC) , 9-Fluorenylmethyloxycarbonyl (FMOC), Acetyl (Ac) group - Acetyl halide, sulfonyl group- p-Toluenesulfonic acid, *p*-toluenesulfonyl chloride, *p*-bromobenzenesulfonyl, 2- or 4-nitrobenzenesulfonyl, methanesulfonic acid (CH₃SO₃H), methanesulfonyl chloride, trifluoromethanesulfonyl, and 5-(dimethylamino)naphthalene-1-sulfonyl.

5. A process for preparation of highly pure Ivabradine hydrochloride according to claim 3 wherein the derivatizing agent is acetyl chloride.

6. A process for the preparation of highly pure Ivabradine hydrochloride comprising step of:
(a) condensing methylamine derivative of formula II with 3-(3-chloropropyl)-7, 8-dimethoxy-1, 3, 4, 5-tetrahydro-2*H*-3-benzazepin-2-one of formula III 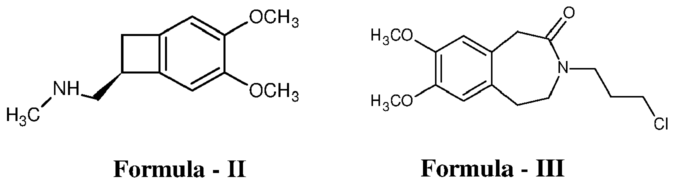 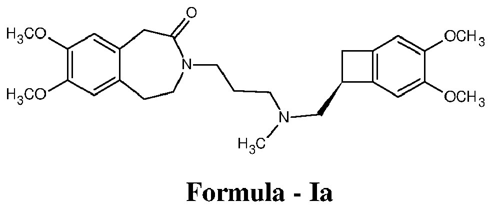
(b) treating crude Ivabradine with acetyl chloride in suitable solvent;
(c) treating Ivabradine with hydrochloride to obtain highly pure Ivabradine hydrochloride;
(d) crystallizing highly pure Ivabradine hydrochloride with acetonitrile and methanol; and
(e) isolating highly pure Ivabradine hydrochloride;

7. A process for preparation of highly pure Ivabradine hydrochloride according to any one of the preceding claims wherein the suitable solvent is selected from the group comprising toluene, tetrahydrofuran, acetone, ethyl acetate, triethylamine, acetonitrile, dimethylformamide, dimethylsulfoxide, water, isopropanol, methylene dichloride, ethylene dichloride, carbon tetrachloride, chloroform, and C₁-C₄ linear aliphatic alcohols and the like or mixtures thereof.

8. A process for preparation of highly pure Ivabradine hydrochloride according to claim 7 wherein the suitable solvent is selected from toluene, triethylamine, dimethylformamide or dimethylsulfoxide or mixtures thereof.

9. Highly pure Ivabradine hydrochloride having a purity of at least about 99 % with the content of N-[(4,5-dimethoxybenzocyclobut-l-yl)-methyl]-7N-(methyl)amine of formula II being in the range of about 0.03 % to about 0.15 % as measured by HPLC.
